# EUROPEAN PATENT SPECIFICATION

(11) **EP 3 204 146 B1**
(45) Date of publication and mention of the grant of the patent: **07.12.2022**
(21) Application number: 15787359.7
(22) Date of filing: 09.10.2015
(51) Int. Cl.: B01D 53/85

(54) **PROCESSES FOR CARBON DIOXIDE CAPTURE WITH BIOCATALYST RECOVERY SYSTEM**
VERFAHREN ZUR KOHLENDIOXIDABSCHEIDUNG MIT EINEM BIOKATALYSATORRÜCKGEWINNUNGSSYSTEM
PROCÉDÉS POUR LA CAPTURE DE DIOXYDE DE CARBONE AVEC UN SYSTÈME DE RÉCUPÉRATION BIOCATALYTIQUE

(30) Priority: 09.10.2014 US 201462061977 P
(43) Date of publication of application: 16.08.2017
(73) Proprietor: SAIPEM S.p.A., 20097 San Donato Milanese (MI) (IT)
(72) Inventor: ZAKS, Aleksey, Québec, Québec G2C 1T9 (CA); REARDON, John, Québec, Québec G2C 1T9 (CA); BUCHOLZ, Tracy, Québec, Québec G2C 1T9 (CA); HULVEY, Matthew, Québec, Québec G2C 1T9 (CA)
(74) Representative: Croce, Valeria
(86) International application number: PCT/US2015/054938
(87) International publication number: WO 2016/057918

(56) References cited:
- WO-A1-2011/076502
- WO-A1-2014/000113
- US-A- 4 382 070
- US-A1- 2012 129 246
- US-A1- 2013 267 004

## Description

### FIELD OF THE INVENTION

The present invention generally relates to processes for the capture of carbon dioxide from gases that are produced by various industrial processes including the capture of CO₂ from flue gases after the combustion of carbon-based fuels. Specifically, the invention relates to processes and systems comprising a biocatalyst recovery system.

### BACKGROUND OF THE INVENTION

Coal is the largest fuel source for the generation of electricity worldwide. World coal consumption is expected to reach 9.05 billion tonnes by 2030. The flue gas generated by coal-fired power plants is the largest contributor to worldwide anthropogenic carbon dioxide, and the capture and sequestration of carbon dioxide produced in the combustion of coal represents an important opportunity to reduce the electric grid's greenhouse gas (GHG) footprint.

Similarly, the combustion of other carbon-based fuel sources, some of which are commonly touted as alternatives to coal, also contribute to GHG emissions. For example, during the period from 1990 to 2012, the combustion of natural gas contributed approximately 24% of the overall GHG emissions associated with the U.S. electric power grid. Flexible and cost effective technologies for the capture of CO₂ are needed. Suitable technologies should be adaptable to a variety of post-combustion sources at near atmospheric pressure that can contain as low as 4% CO₂ to as high as 16%> CO₂, depending on plant efficiency, fuel source, and excess combustion air requirements. Also, there are opportunities to remove CO₂ from biomass fermentation and digestion processes at near ambient pressure which may have up to 40% CO₂ by volume.

US 2012/129246 A1 and WO 2011/076502 A1 disclose a process for removing CO₂ from a CO₂-containing gas, with a biocatalyst comprising a carbonic anhydrase. US 2013/267004 A1 discloses flotation as one suitable method of separating particles to which carbonic anhydrase is bonded from a rich absorption solution which subsequently is to be regenerated.

A need exists for an efficient, economical process for capturing carbon dioxide from gases produced from industrial sources. In particular, a need exists for an efficient, economical process for capturing carbon dioxide from flue gas produced in a coal-fired or natural gas fired power plant.

Accordingly, a process providing efficient carbon capture is needed that can meet the CO₂ removal requirements of industrial applications.

### SUMMARY OF THE INVENTION

The present invention relates to a process according to claim 1 and dependent claims thereto.

### DESCRIPTION OF THE FIGURES

Figure 1 is a process flow diagram of a carbon capture system having a biocatalyst recovery system.
Figure 2 is a process flow diagram of a biocatalyst recovery system including a bulk filter array with sequenced drain and back flush of the filter with lean solution.
Figure 3 is a process flow diagram of a biocatalyst recovery system including a cross-flow filter.
Figure 4 is a process flow diagram of a biocatalyst recovery system including a settling tank.
Figure 5 is a bar graph of the increase in cost of electricity (COE) for a supercritical coal fired power plant with carbon capture comparing various systems.
Figure 6 is a graph of the specific reboiler heat duty (GJ/t CO₂) as a function of the biocatalyst particle concentration (35 wt% KDMG, XC,Rich = 0.8 mol/mol) with two limiting case applications (lean loadings of 0.225 mol/mol for LNG and 0.40 for flue gas), and various rich solution carry over specifications.
Figure 7 is a graph of the normalized energy penalty as a function of the biocatalyst particle concentration (35 wt% KDMG, XC,Rich = 0.8 mol/mol) with various rich solution carry over specification targets.
Figure 8 is a graph of the absorbance versus time for a buoyancy (floatation separation) experiment that quantifies the progress of clarification by light absorbance over time, for various solution temperatures.
Figure 9 is a graph of the typical particle size distributions (percent mass of particles with diameter) for various biocatalyst samples.
Figure 10 is a graph of the absorbance versus time for a buoyancy (floatation separation) experiment that quantifies the progress of clarification by light absorbance over time at 70°C.

Corresponding reference characters indicate corresponding parts throughout the drawings.

### DESCRIPTION OF THE INVENTION

It has been discovered that economic and continuous recovery of biocatalyst particles from a rich solution (e.g., a high concentration of bicarbonate salts after CO₂ capture) by quantitatively transferring the biocatalyst particles into an equivalent flow of lean solution (e.g., a low concentration of bicarbonate salts after solvent regeneration) with minimal rich solution carry over in the wet biocatalyst is advantageous. Minimizing rich solution carry over is important to maintaining the most favorable energy balance in lean critical CO₂ capture applications where deep CO₂ removal is required, and may include either high pressure or near ambient pressure applications where a low CO₂ partial pressure is required in the treated gas regardless of the extent of CO₂ removal.

Biocatalyst properties including increasing the concentration of the biocatalyst particles near the gas-liquid interface have been shown to improve biocatalyst efficiency, meaning a smaller concentration of the biocatalyst is required for the desired CO₂ capture application. In addition, the biocatalyst properties that drive efficient CO₂ hydration catalysis also promote more rapid separation of the biocatalyst particle via floatation, enabling a smaller and lower cost biocatalyst recovery system. The efficiency of the biocatalyst particles works together with the efficient biocatalyst recovery system to minimize rich solution carry over and provides the lowest energy solvent regeneration and best economics of the biocatalyst enhanced CO₂ capture system.

The most important aspect of the BRS are (1) it minimizes rich solution carry over (grams rich solution per gram biocatalyst recovered), (2) it operates continuously (including continuous rich feed and steady biocatalyst product), (3) it provides stable and repeatable long-term performance, (4) it minimizes size, cost and complexity of the BRS equipment, and (5) it is simple to operate with minimal maintenance outage requirements.

The invention can be directed to a system for removing CO₂ from a CO₂-containing gas comprising a scrubbing vessel having (i) a bottom portion containing a gas inlet and a liquid outlet to release CO₂-rich liquid; (ii) a top portion containing a liquid inlet for CO₂-lean liquid, and a gas outlet for CO₂-lean gas, and (iii) a middle portion containing a plurality of biocatalyst particles, a biocatalyst recovery unit comprising a solid/liquid separator, an outlet for the CO₂-rich liquid, and an outlet for the biocatalyst solids. For this system, the biocatalyst recovery unit operates continuously by separating the biocatalyst particles from the CO₂-rich liquid.

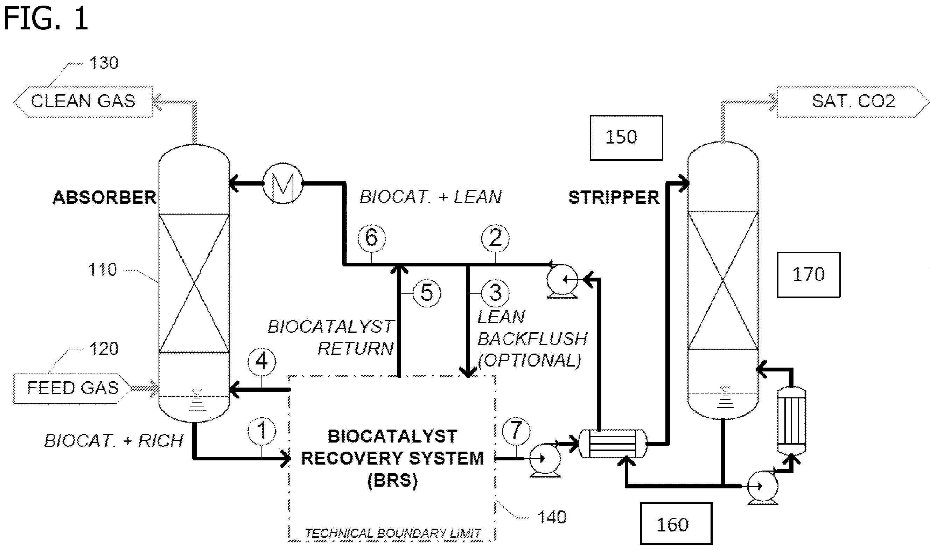

The generic integration of the "biocatalyst recovery system" (BRS) into the overall biocatalyst enhanced CO₂ capture process is illustrated in Figure 1. In Figure 1, an absorber 110 has a feed gas inlet 120, a clean gas outlet 130 and a bottoms stream 1 containing the biocatalyst and a rich liquid (e.g., a high concentration of bicarbonate salts). The bottom stream 1 ran from the bottom of the absorber 110 into the biocatalyst recovery system (BRS) 140. The biocatalyst was separated from the rich solution in the BRS 140 and the biocatalyst was returned to the absorber 110 using the biocatalyst return stream 5 and the rich solution stream 7. The rich stream 7 was fed to the shipper 170 using the rich transfer line 150. Lean solution 160 exited the bottom of the stripper 170 and was pumped in the lean solution stream 2 to connect with the biocatalyst return stream 5 that added the biocatalyst to the lean solution in the biocatalyst and lean solution stream 6. The volume flow of the lean solution stream 2 is approximately equal to the flow of rich solution stream 7. The biocatalyst and lean solution was pumped to the top of the absorber 110. The lean solution stream 2 can also be used as a lean backflush 3 for the biocatalyst recovery system 140. For options that require a lean solution back- flush to recover the biocatalyst wet-cake, a rich solution drain or purge step is required in BRS operating sequence to minimize rich solution carryover.

In Figure 1, a rich solution stream containing an approximately 1 % w/w suspension (0.5% to 3% w/w as needed) of biocatalyst is produced in the absorber 110 after capturing CO₂ from a feed gas stream. Rich solution containing suspended biocatalyst particles is discharged in the absorber bottom stream 1 where it is subsequently pumped to the BRS.

Also in Figure 1 , a lean solution is received from the regeneration system in the lean solution stream 2. For example, two or more barrier filters that operate with back-pulse or back flush from time to time to release the wet biocatalyst filter cake, and by using lean solution rather than rich solution, the biocatalyst could be transferred from rich solution to lean solution with minimal rich solution carry over. Therefore, the rich solution is discharged from the filter housing at stream 4 before a lean back-flush stream 3 is supplied. Rich solution stream 4 is directed back to the absorber 110 bottom reservoir.

For the system depicted in Figure 1, the concentrated biocatalyst particle suspension (or biocatalyst retentate) in stream 5 shall have minimal rich solution carry over (e.g., less than 7 g rich solution/gram biocatalyst (dry, salt free basis), and preferably less than 5 g/g). Moreover, the particle suspension in lean solution 5 returned to the process stream can be delivered on a continuous basis and at a constant particle concentration to provide steady state dilution blending with additional lean solution to produce the desired biocatalyst particle concentration in the lean solution stream 6.

The biocatalyst concentration in the lean solution is approximately 0.1 %w/w to 3%w/w, 0.1%w/w to 2.5%w/w, 0.1%w/w to 2%w/w, 0.5%w/w to 2%w/w, 0.5%w/w to 1.5%w/w, or 1% w/w.

The rich solution permeate stream 7 of Figure 1 shall be substantially free of biocatalyst particles when discharged from the BRS after which it is pumped to the CO₂ stripping, solvent regeneration system, that operates at elevated temperatures.

Generally, the biocatalyst recovery system can provide a way to separate the biocatalyst from the rich liquid medium by filtering the biocatalyst.

The filtering of the biocatalyst can further comprise a back flush of the filter with the lean liquid medium to produce the separated biocatalyst.

The filtering of the biocatalyst can be performed using a back-pulse or back-flush filter array, a cross flow membrane, a flow-through membrane, a sintered metal filter, a single media filter, a multimedia filter, or a combination thereof.

The filtering of the biocatalyst can be performed using a single media filter or a multimedia filter.

Generally, various types of filter configurations for solid/liquid filtration can be used. These filter configurations include (1) outside-in filtration where a traditional solid/liquid barrier separation occurs on the outer perimeter of a tubular filter element, (2) inside-out filtration where a solid/liquid barrier separation occurs on the inside of a tubular filter element, (3) inside-out (multimode) filtration where a solid/liquid (barrier or crossflow) separation that occurs on the inside of open-ended tubular filter element and filtration is with multi-option top or bottom feed inlet.

Further, the biocatalyst can be separated from the rich liquid medium using a gravity-assisted separation technique.

The gravity-assisted separation technique can include a conventional jig, a spiral, a pinched sluice, a centrifugal jig, a centrifugal concentrator, a wet shaking table, a dry shaking table, a Mozeley gravity separator, or a combination thereof.

The separation of the biocatalyst is performed with about 90%, about 91%, about 92%, about 93%, about 94%, about 95%, about 96%, about 97%, about 98%, about 99%, by weight separation efficiency.

Additionally, the separated biocatalyst has less than about 7 gram, less than about 6 gram rich liquid medium per gram biocatalyst based on a dry, salt free biocatalyst.

The process of any one of claims 1 to 10 wherein the lean liquid comprises about 0.1 to about 3 wt.% biocatalyst.

### Biocatalyst Recovery Systems (BRSs)

A number of systems can be used for the BRS including: (a) a bulk filter array with a sequenced drain and a back-pulse/back- flush with a lean solution, (b) a cross-flow membrane system, and (c) a floatation tank with skimmer and simplified fines polishing filter with rich solution back- flush. The floatation tank system can be used with biocatalyst particles of apparent low density that achieves rapid floatation separation and reduces the size of the vessel required for floatation skimming. Also notable in this configuration is that fines recovery to the floatation system is simplified by using rich solution back flush.

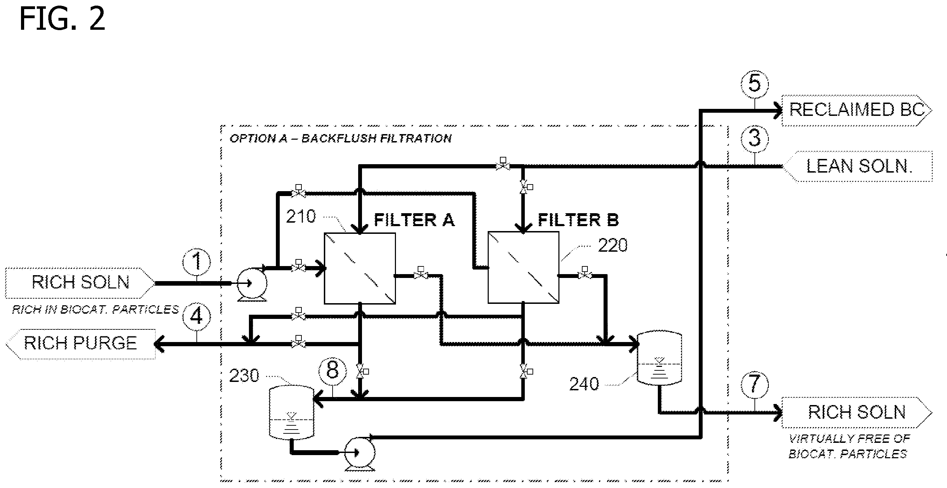

Figure 2 is a process flow diagram of the BRS-A system. One advantage of the BRS-A system is a dense wet-cake is formed and thus, rich solution carry over is minimized. For example, preliminary filtration tests suggest rich solution carry over for this BRS-A system will be less than 5 g/g biocatalyst. The system also is expected to be robust and require minimal off-line filter maintenance, while using valve sequencing and surge tanks to deliver continuous flow of biocatalyst product.

In BRS-A, the rich solution stream 1 enters the BRS and contacts two (or more) barrier filters, Filter A 210 and Filter B 220, arranged in parallel to separate the biocatalyst particles from the rich solution. In typical operation, both filters can operate to collect biocatalyst particles from rich solution feed, or one filter can be isolated on periodically for biocatalyst recovery- which can be determined based on a predetermined cycle time or based on a filter pressure drop trigger. The biocatalyst recovery sequence would include a drain step to purge rich solution at stream 4 followed by a lean fill and possible back pulse then back flush using lean solution from stream 3. For example, after a period of time Filter A 210 will have a full wet cake on its internal filter elements. At that time valves will isolate Filter A 210 while maintaining a constant rich solution stream 1 by directing the full flow to Filter B 220 (and others as required). Next, the rich solution will be drained from the housing of Filter A 210 and thus purged via rich purge stream 4 to be recycled to the absorber bottom tank. Afterward, lean solution stream 3 will fill the spare volume of the Filter A 210. Lean solution stream 3 can include a pulsing step to efficiently release the wet cake into the filter tank volume. Finally a reverse flow (or back flush) from lean solution stream 3 will carry the recovered biocatalyst wet cake to an accumulation tank via stream 8. Finally, Filter A 210 will be isolated again, and then returned to filtration process function by rich solution stream 1 filling the vessel with rich solution (and displacing any intake gases). The biocatalyst wet cake recovery would be repeated on Filter-A 210 and Filter-B 220 as needed. A similar process occurs when Filter B 220 requires regeneration. The filtrate from both filters enters an accumulation tank and is then pumped for regeneration through rich solution stream 7. Retentate from both filters flows through stream 8 to an accumulation tank 230 and the biocatalyst is pumped through stream 5 back to the top of the absorber.

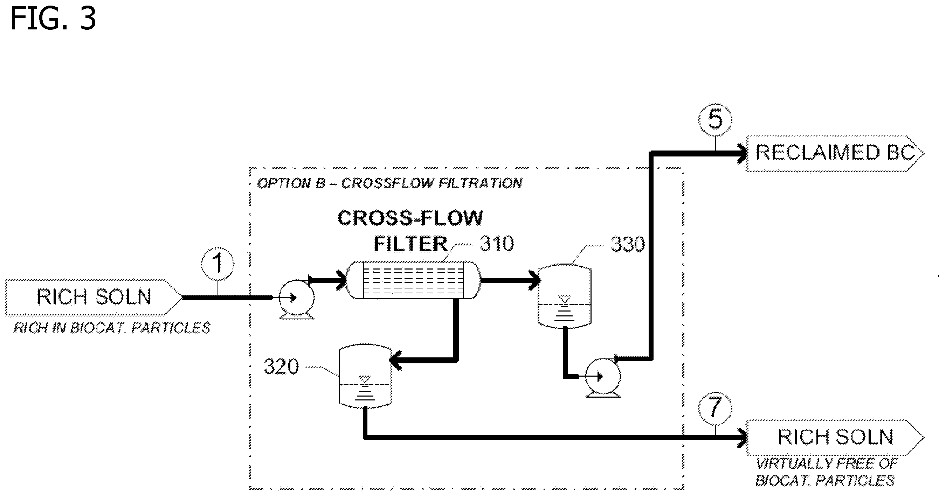

A process flow diagram of the BRS -B system is shown in Figure 3. One advantage of the BRS-B system is that it has the simplest continuous process configuration. A continuous rich solution stream 1 enters at one end of the tube side of the cross-flow filter 310 having a membrane and the slurry of rich solution and biocatalyst becomes progressively concentrated before being discharged at the other end of the cross-flow filter 310. A continuous clean rich solution stream 7 is produced on the shell side of the cross-flow filter membrane unit 310.

Preliminary membrane tests demonstrated continuous production of a dense biocatalyst sludge having rich solution carry over less than 5 g/g biocatalyst and a continuous liquid flux greater than about 80 gallons/square ft/day.

A rich solution stream 1 of the BRS-B system is pumped to a suitable pressure required for the desired rich solution flow rate. The BRS-B is comprised of a cross-flow filter membrane unit 310, or an array of cross-flow filter membrane units operating in parallel or in series-parallel configuration depending on the particular design. Biocatalyst concentrate flows into an accumulation tank 330 where metering pumps would inject reclaimed biocatalyst stream 5 into the lean solution stream 2 on a continuous basis and at the rate required for the desired concentration (e.g., 1% w/w) needed at the CO₂ absorption unit. Clean rich solution, or permeate, collects in another accumulation tank 320 before being discharged in the rich solution stream 7, where it delivered for thermal regeneration.

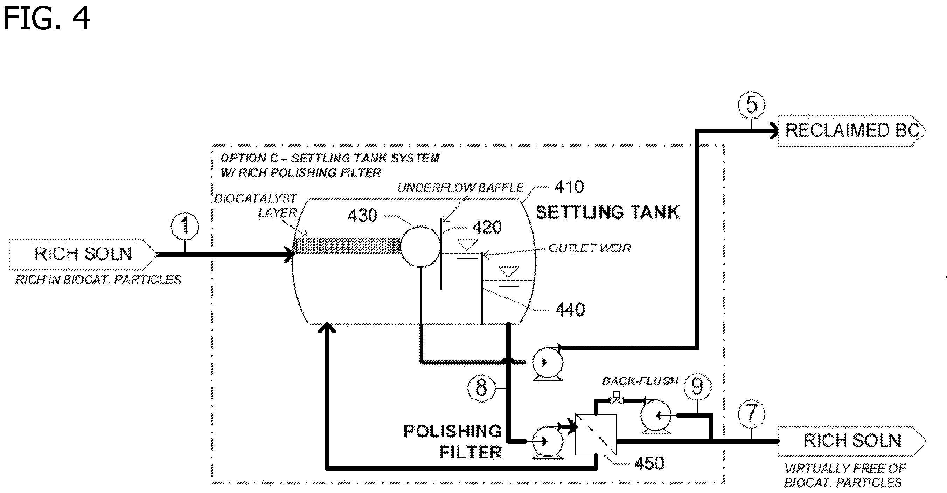

A process flow diagram of the BRS-C system is shown in Figure 4. The BRS-C system capitalizes on the biocatalyst particle's physical and chemical character that provides rapid rise of the biocatalyst particles to the liquid surface. The density of the wet cake may depend on a given formulation, settling time, and concentration in the floatation tank. Process parameters can be configured to minimize rich solution carry over.

One advantage of the BRS-C system is the simplicity of passive separation in the floatation tank. This separation could be performed within the absorber bottom reservoir. Also, the rich solution polishing filter does not need complex drain and purge sequences because it can be back flushed with rich solution and then recycled to the floatation tank. The BRS-C system could be robust and require minimal off-line maintenance and could likely be able to produce a continuous flow of biocatalyst particles and continuous flow of clean rich solution.

In the BRS-C system shown in Figure 4, the rich solution stream 1 enters the BRS-C and flows into a settling tank 410 with enough residence time to allow bulk separation of the biocatalyst particles (e.g., mean residence time of 5 to 10 minutes). An underflow baffle 420 is installed where biocatalyst particles collect in a floating layer to facilitate collection in a skimmer device 430. Recovered biocatalyst would be continuously pumped through reclaimed biocatalyst stream 5 to mix with the lean solution stream 2 to the concentration desired in the CO₂ absorption unit.

Rich solution flows beneath the underflow baffle where it spills over an outlet weir 440 that fixes the liquid level in the settling tank 410. Rich liquid collects in a spill over after which it is pumped in rich solution stream 8 into a fines polishing filter 450. The fines polishing filter 450 removes the remaining biocatalyst particles from the rich solution stream 7. In this case, rich solution stream 9 can be used as needed to provide back-pulse/back-flush to release fines back to the floatation tank.

The separation by flotation can be facilitated by introducing gas microbubbles into the rich liquid medium containing biocatalyst particles. The gas introduced can be air, nitrogen, argon, or another gas that is effective for forming microbubbles to enhance the separation of the biocatalysts by floatation. The parameters used for the gas introduction depend on the particular separation system used.

A particular flotation separation method is dissolved air flotation (DAF). This method provides air or gas bubbles that aid the separation of the biocatalysts from the rich liquid medium.

Lean pinch in the solvent regeneration system (re-boiler and stripper column) is an important issue impacting energy balance where rich solution recycle is used. Lean pinch is more significant when the solvent to be regenerated becomes leaner. Rich carry over to the lean solution with recycled biocatalyst can be offset by more extensive steam stripping (more lean than otherwise required) in the solvent regeneration system. The amount of rich solution carry over depends on the BRS technology used. Applications that require a leaner solution to achieve deeper CO₂ removal will have more energy output with rich solution recycle. However, if rich solution carry over can be minimized then the energy impact can be acceptable or virtually eliminated in some cases.

Two limiting CO₂ capture applications were studied based on the lean loading requirement needed by the application. Biogas treating is estimated to require a 0.4 mol/mol lean loading with potassium dimethyl glycine (KDMG). By contrast, gas treating for LNG specification will require a 0.22 mol/mol lean loading. Both applications are assumed to be able to achieve a rich loading of 0.8 mol/mol with KDMG. Flue gas applications will fall somewhere between these limiting cases, but more closely approximated by the biogas application.

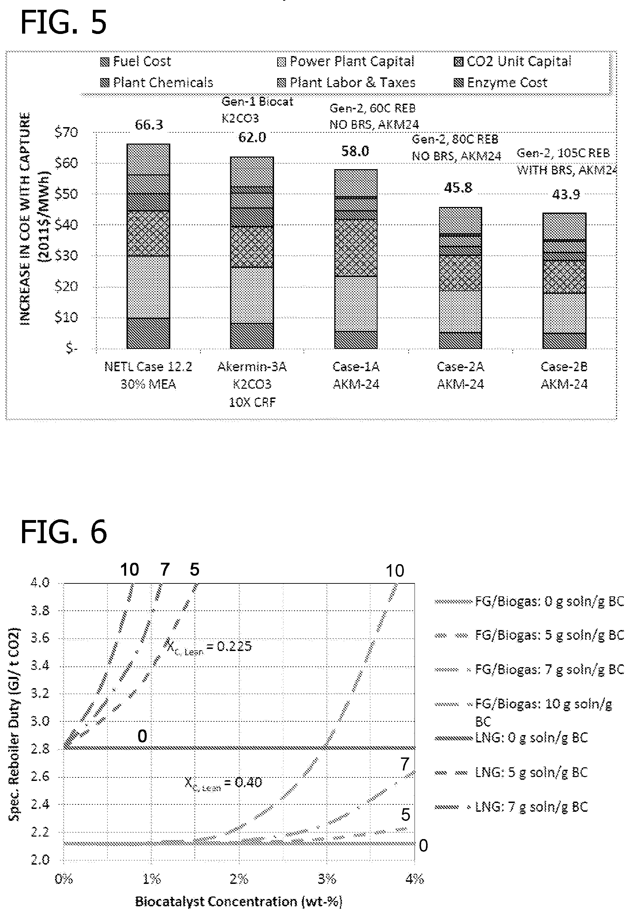

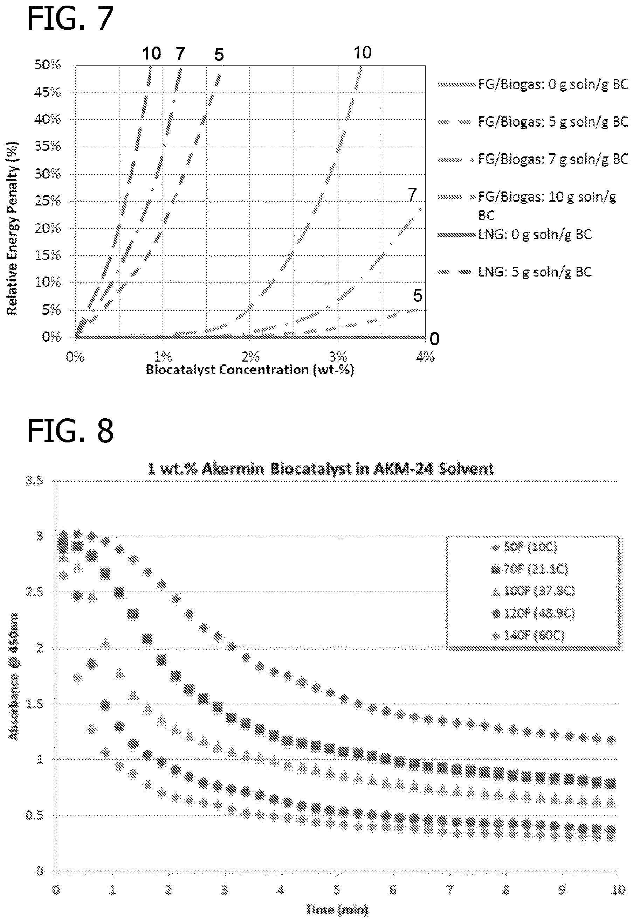

Aspen simulation was used to predict the energy requirement for each application with rich solution recycle including 0, 5, 7, and 10 g/g solvent/biocatalyst assuming various biocatalyst efficiencies (biocatalyst concentration required for the given absorption application). Figure 6 presents the specific reboiler duty as a function of biocatalyst particle concentration, and Figure 7 follows with the normalized energy penalty relative to a system without rich solution recycle.

Simulation results showed operating boundaries for each application. The biocatalyst can be added as needed (to off-set declining performance with time, for example) until a maximum biocatalyst concentration is reached. The maximum concentration could be defined based on practical considerations of particle mixture or when regeneration energy penalty starts exceeding acceptable threshold (for example, not more than 10% over the nominal regeneration energy).

The BRS requirements can be more flexible for the biogas application or a similar application where nominal lean loading is 0.4 mol/mol, because lean pinch is less important and energy penalty for regenerating to leaner conditions becomes less significant. For example, if the biocatalyst concentration were 3% and the BRS rich carry over performance was 7 g/g biocatalyst, then the energy penalty would be about 7%.

In contrast, the BRS rich carry over requirement and maximum biocatalyst concentration are stricter for the deep CO₂ removal applications like LNG. For example, if the biocatalyst concentration was 1% and the BRS rich carry over performance was 7 g/g, then the energy penalty would be about 34%. Or, if BRS rich carry over specification can be held to a tighter performance standard such as 5 g/g, then the energy penalty would be 20%>.

The maximum rich carry over specification for the BRS is 7.0 g rich solution/g biocatalyst. Preferably, the rich carry over performance should be better than 5 g /g biocatalyst. Also, the minimum filtration efficiency is recommended to be 99.95% or better. These minimum performance specifications are summarized in Table 1 below.

**Table 1: Recommended performance requirements for BRS**

| Process Parameter | System |
|---|---|
| Minimum Filtration Efficiency @ >5 µm | >99.95% |
| Preferred (Design) Rich Carryover at (5) | 5.0 g/g |
| Maximum Carryover at (5) | <7.0 g/g |

The economic merits of various systems are presented in Figure 5, comparing the cost of electricity with CO₂ capture for 550 MWe net super-critical coal fired power boiler application (15.9% CO₂, dry basis) with various CO₂ capture systems. Notably, the Gen-2 biocatalyst system with BRS (105°C reboiler eliminates vacuum equipment) achieves >30% reduction in cost of CO₂ capture relative to NETL Case-12.

### Biocatalyst

Generally, the biocatalyst can be an immobilized enzyme, a ribozyme, a deoxyribozyme, an enzyme mimic, or an organic or inorganic bio-mimetic compound that can catalyze the hydration of carbon dioxide and/or the dehydration of bicarbonate.

The biocatalyst particles can comprise a composite structure having hydrophobic characteristics that retain gas pockets within micropores provides an apparent low density, and this low density and hydrophobic character enhances rapid separation of the particles from solution (i.e., floatation separation). This separation is further enhanced with increased solution temperatures.

The development of a floating wet-cake after one minute at rest using a more recent biocatalyst formulation can be observed when the carbonic anhydrase xerogel particles are dispersed in water. After several minutes there was a continued clarification. For comparison, the liquid in the absorber bottom reservoir typically has a five minute or longer residence time, which is sufficient for formation of a floating wet cake.

### Biocatalyst Delivery

Alternatively, the biocatalyst may spend a disproportionate amount of time at the gas-liquid interface as compared to being immersed deeper within the bulk liquid medium. The properties and design of the biocatalyst micro-particle, including the density and degree of surface hydrophobicity, may be selected to provide a disproportionate surface concentration.

In these processes and methods, the use of a biocatalyst micro -particle (for example, active carbonic anhydrase enzymes that are entrapped within a micro-particulate supporting structure) provides a number of advantages as compared to the use of a biocatalyst that is directly dissolved within the liquid medium (e.g., a soluble enzyme). For example, soluble enzymes are known to inactivate easily under high shear conditions presented by high pressure pumps, high velocity flow through pipes, flow over high gas-liquid surface area packed columns, flow through gas sparged contactors, and especially locations with rapid degassing and boiling that are typical of the solvent regeneration system (e.g., in the stripper and reboiler). Moreover, denatured enzymes can contribute to foaming upsets and a range of significant process-related issues throughout the absorber, stripper, and reboiler. Since the denaturing is typically accelerated under degassing conditions, a soluble enzyme delivery will result in untenable operational issues.

To address these issues, the biocatalyst may be immobilized by entrapment within a solid particulate material or covalently bonding the biocatalyst to the solid material.

For example, the biocatalyst may be delivered in the liquid medium as a plurality of nanoparticles or microparticles. Each biocatalyst particle may be comprised of a protective structure that encapsulates the biocatalyst, but also provides un-hindered diffusion of CO₂ and bicarbonate ions into and out of the areas of the particle that contain the active catalyst.

The active biocatalyst particles can form a free-flowing suspension in the liquid medium.

Typically, the sol-gel process for creating enzyme-containing xerogel particles begins with a solution containing the biocatalyst, alkoxysilane monomers, and any additives and solvents necessary for the given formulation. Upon addition to an aqueous solution, and usually with the aid of a polymerization catalyst, the monomer molecules begin to hydrolyze and form bonds with other monomer molecules, eventually forming a colloidal suspension of micro-particles (a "sol"). The condensation of the sol into a gel phase results in the biocatalyst being entrapped within the polymeric matrix, forming stabilizing pores in the material. As polymerization proceeds and solvent is evaporated, a gel is condensed, and later becomes a dried organo-silicate biocatalyst material containing the encapsulated biocatalyst.

For example, the xerogel particles can be formed by spray-drying the material into a fine powder in heated air, by drying the bulk gel and mechanically grinding and sieving to an appropriate particle size, by forming the gel in a non-solvent via a micro -emulsion process, by CO₂ supercritical drying, or by any other means known in the art.

For example, the biocatalyst may comprise a xerogel particle derived from a sol comprising (i) an alkoxy silane or an organotrialkoxy silane or metasilicate, (ii) a poly(silicone), and (iii) a biocatalyst. The biocatalyst xerogel particle typically has a particle size range of 100 nm to less than 10 µιη or more than 20 µιη to 250 µιη.

The size of the primary particles, the pore diameter, the size of the agglomerated clusters, and the surface charge are expected to affect the performance of the biocatalyst particles in liquid suspension. As a result, control of these features during production is desirable, and as such a reproducible production method is typically employed.

Typically, the particle is derived from reaction of a sol and a catalyst.

For example, the biocatalyst may be an immobilized enzyme comprising an enzyme and an immobilization material, wherein the enzyme is entrapped within the immobilization material and the immobilization material is derived from reaction of a sol and a catalyst, the sol comprising (i) an alkoxy silane or an organotrialkoxy silane or metasilicate, (ii) a poly(silicone), and (iii) an enzyme. Typically, the poly(silicone) comprises a poly(siloxane).

Generally, when the biocatalyst is immobilized by entrapment within an immobilization material, the immobilization material may have an overall pore volume, surface area, and/or average pore size as described above.

When the biocatalyst is immobilized (e.g., in a xerogel particle as described above), the immobilization material typically has an overall pore volume of at least about 3 µL/g to 500 µL/g.

The immobilization material may have a surface area of at least about 1 m²/g, at least about 5 m²/g, at least about 10 m²/g, at least about 20 m²/g, at least about 30 m²/g, at least about 40 m²/g, at least about 50 m²/g, at least about 60 m²/g, at least about 70 m²/g, at least about 80 m²/g, at least about 90 m²/g, at least about 100 m²/g, at least about 150 m²/g, at least about 200 m²/g, or at least about 300 m²/g.

For example, the immobilization material typically has a surface area of from about 1 m²/g to about 400 m²/g, from about 5 m²/g to about 300 m²/g, from about 10 to about 150 m²/g, or from about 15 to about 100 m²/g.

The immobilization material may also have an average pore size of from about 2 nm to about 80 nm.

Further, the biocatalyst can be a carbonic anhydrase xerogel particle comprising carbonic anhydrase entrapped within a xerogel particle, the carbonic anhydrase xerogel particle being derived from a sol comprising (i) an alkoxy silane, an organotrialkoxy silane, or a metasilicate, (ii) a hydrophobic organotrialkoxy silane, a hydrophobic organohalosilane, or a combination thereof, (iii) a poly(silicone), (iv) a hydrophilic additive, and (v) carbonic anhydrase.

Further, the carbonic anhydrase xerogel particle can have the hydrophobic organotrialkoxy silane or hydrophobic organohalosilane comprise an alkyl chlorosilane, a fluoroalkyl chlorosilane, a phenyl chlorosilane, an alkyl trialkoxysilane, a fluoroalkyl trialkoxysilane, a phenyl trialkoxysilane, or a combination thereof.

Additionally, the carbonic anhydrase xerogel particle can have the hydrophobic organotrialkoxy silane or hydrophobic organohalosilane comprise octadecyldimethylchlorosilane, benzyldimethyl chlorosilane, octadecyl trimethoxysilane, phenylethyl trimethoxysilane, nonafluorohexyl trimethoxysilane, (3,3,3-trifluoropropyl)dimethyl chlorosilane, isobutyl trimethoxysilane, nonafluorohexyldimethyl chlorosilane, tridecafluoro-1 , 1 ,2,2-tetrahydrooctyl)dimethyl chlorosilane, (heptadecafluoro- 1 , 1 ,2,2-tatrahydrodecyl)dimethyl chlorosilane, or a combination thereof.

Also, the carbonic anhydrase xerogel particle can have the hydrophobic organotrialkoxy silane comprise nonafluorohexyl trimethoxysilane, isobutyl trimethoxysilane, or a combination thereof.

Further, the carbonic anhydrase xerogel particle can have the alkoxy silane comprise tetramethylorthosilicate, tetraethylorthosilicate, methyltriethylorthosilicate, ethyltrimethylorthosilicate, dimethyldiethylorthosilicate, tetraglyceryl silicate, or a combination thereof.

The carbonic anhydrase xerogel particle can have the organotrialkoxy silane be trimethoxymethylsilane, trimethoxyethylsilane, or a combination thereof.

Also, the carbonic anhydrase xerogel particle can have the alkoxy silane comprise tetramethylorthosilicate .

The carbonic anhydrase xerogel particle can have the poly(silicone) be a poly(siloxane) selected from the group consisting of poly(dimethylsiloxane), poly(dimethylsiloxane)-co-poly(alkene oxide), or a combination thereof.

The carbonic anhydrase xerogel particle can have the poly(siloxane) comprise polydimethylsiloxane.

The carbonic anhydrase xerogel particle can have the poly(silicone) be silanol-terminated.

The carbonic anhydrase xerogel particle can comprise an alkoxysilane of tetramethyl orthosilicate, a poly(silicone) of a silanol-terminated poly(dimethyl siloxane), a hydrophilic additive of a crown ether, and a hydrophobic organotrialkoxy silane of isobutyl trimethoxysilane, nonafluorohexyl trimethoxysilane, or a combination thereof.

Biocatalyst powders can be produced using spray drying technology.

A typical particle size distribution of the biocatalyst particles can have an average particle size in the range of from about 5 µιη to about 100 µιη;

Further, the biocatalyst particles can have an average particle size in the range of from about 10 to 50 µιη.

### Biocatalyst

As described in detail above, the biocatalyst can comprise enzyme catalysts that are incorporated into a particle delivery system.

Alternatively, the biocatalyst can include known organic catalysts, inorganic catalysts, or bio-mimetic catalysts that are incorporated into a particle delivery system.

When the biocatalyst comprises an enzyme, naturally-occurring enzymes, man-made enzymes, artificial enzymes and modified naturally-occurring enzymes can be utilized. In addition, engineered enzymes that have been engineered by natural or directed evolution can be used. Also, an organic or inorganic molecules that mimics an enzyme's properties can be used.

### Carbonic Anhydrase

One example of a biocatalyst is carbonic anhydrase. Carbonic anhydrase accelerates CO₂ hydration by catalyzing de-protonation of water, shuttling the proton away from the active site, and converting CO₂ at the active site to bicarbonate ion with a characteristic reaction time of approximately 10⁻⁶ seconds. The reversible dehydration of bicarbonate is also catalyzed by carbonic anhydrase, as shown below.

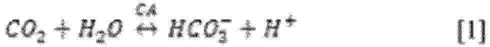

Without being bound by theory, it is believed that a carbonic anhydrase enzyme catalyzes hydration of carbon dioxide by having a zinc atom in the active site that coordinates to three histidine side chains while having the fourth coordination position of the zinc atom occupied by water. The coordination of the water by the zinc atom causes polarization of the hydrogen-oxygen bond. A fourth histidine accepts a proton from the coordinated water molecule resulting in a hydroxide attached to the zinc atom. The carbonic anhydrase active site also contains a pocket for carbon dioxide that brings it close to the hydroxide group and allows the electron-rich hydroxide to attack the carbon dioxide to form bicarbonate. In this way, the carbonic anhydrase is involved in the hydration of carbon dioxide. (Tripp, B. C, Smith, K., & Ferry, J. G. (2001). Carbonic Anhydrase: New Insights for an Ancient Enzyme. Journal of Biological Chemistry, 276 (52), 48615-48618.)

Generally, the term carbonic anhydrase ("CA") represents a family of structurally and genetically diverse enzymes that arose independently from different precursors as a result of convergent evolution (Tripp, B. C, Smith, K., & Ferry, J. G. (2001). Carbonic Anhydrase: New Insights for an Ancient Enzyme. Journal of Biological Chemistry, 276 (52), 48615-48618; Elluche, S., & Poggeler, S. (2010). Carbonic Anhydrases in Fungi. Microbiology, 156, 23-29). The various CA enzymes have been organized into five unrelated structural classes (e.g., alpha, beta, gamma, delta, and epsilon) which share no DNA sequence similarity and differ in protein structure and active site architecture. Despite these structural differences, the active sites of all classes of CA enzymes function with a single divalent metal cofactor which is essential for catalysis (Tripp, B. C, Smith, K., & Ferry, J. G. (2001). Carbonic Anhydrase: New Insights for an Ancient Enzyme. Journal of Biological Chemistry, 276 (52), 48615-48618). The most common metal cofactor in CA enzymes is zinc.

The a-class of CA is the predominant form expressed in mammals, and is the best characterized of all the CA classes. There are at least 16 a-CA or CA-related enzymes (Supuran, C. T. (2008). Carbonic Anhydrases - An Overview. Current Pharmaceutical Design, 14, 603-614 found in animals, as well as six forms found in bacteria. The β-class of CAs are found in green plants, blue-green algae, and bacteria (Zimmerman, S. A., & Ferry, J. G. (2008). The β and γ Classes of Carbonic Anhydrases. Current Pharmaceutical Design, 14, 716-721) (Rowlett, R. S. (2010). Structure and Catalytic Mechanism of the β-Carbonic Anhydrases. Biochimica et Biophysica Acta, 1804, 362-373). The γ-class is found in bacteria and an example would be the CA from Methanosarcina thermophila (CAM) (Zimmerman, S. A., & Ferry, J. G. (2008). The β and γ Classes of Carbonic Anhydrases. Current Pharmaceutical Design, 14, 716-721). The CAM gene has been cloned into E. coli and is expressed as the Zn-containing form (Alber, B. E., & Ferry, J. G. (1996). Characterization of Heterologously Produced Carbonic Anhydrase from Methanosarcina thermophila. Journal of Bacteriology (June), 3270-3274), but it is more active as the Fe-, Cd-, or Co-form. The δ-class can be found in the marine diatom Thalassiosira weissflogii (Zimmerman, S. A., & Ferry, J. G. (2008). The β and γ Classes of Carbonic Anhydrases. Current Pharmaceutical Design, 14, 716-721). This example protein is a dimer, with a monomeric molecular weight of 27 kD. The protein will bind Zn-, but Fe- and/or Cd-predominates in vivo. Likewise, the ζ-class is also found in the marine diatom Thalassiosira weissflogii (Zimmerman, S. A., & Ferry, J. G. (2008). The β and γ Classes of Carbonic Anhydrases. Current Pharmaceutical Design, 14, 716-721). The protein is also a dimer with a molecular weight of 50-60 kD. The catalytic properties of these two classes have not been characterized.

The mammalian CA enzymes are divided into four broad subgroups depending on the tissue or cellular compartment location (e.g., cytosolic, mitochondrial, secreted, and membrane-associated). The CAII and CAIV enzymes are the most catalytically efficient of all the CAs characterized, demonstrating rates of catalysis that are near the theoretical limit for diffusion-controlled rates. CA IV demonstrates particularly high temperature stability, which is believed to result from the presence of two disulfide linkages in the enzyme.

Mammalian carbonic anhydrase, plant carbonic anhydrase, or microbial carbonic anhydrase; preferably, bovine carbonic anhydrase II or human carbonic anhydrase IV is used. Human carbonic anhydrase IV is available from William S. Sly at St. Louis University and is described in more detail in the following references: T. Okuyama, S Sato, X. L. Zhu, A. Waheed, and W. S. Sly, Human carbonic anhydrase IV: cDNA cloning, sequence comparison, and expression in COS cell membranes, Proc. Natl. Acad. Sci. USA 1992, 89(4), 1315-1319 and T. Stams, S.K. Nair, T. Okuyama, A. Walleed, W.S. Sly, D. W. Christianson, Crystal structure of the secretory form of membrane-associated human carbonic anhydrase IV at 2.8 -A resolution, Proc. Natl. Acad. Sci. USA 1996, 93, 13589-13594.

Compounds that mimic the active site of carbonic anhydrase can also be used. For example, various metal complexes have been used to mimic the carbonic anhydrase active site. For example, [Zn₂(3,6,9,12,20,23,26,29-octaazatricyclo[29.3.1.1^{14,18}]hexatriaconta-1(34), 14,16,18(36),31(35),32-hexaene)(CO₃)]Br₂-7H₂O and [Zn₂(3,6,9,12,20,23,26,29-octaazatricyclo[29.3.1.1^{14,18}]hexatriaconta-1(34), 14,16,18(36),31(35),32-hexaene)(CO₃)]Br₂ 0.5CH₃COCH₃-5H₂O (See Qi et al., Inorganic Chemistry Communications 2008, 11, 929-934). Also used as a mimic for carbonic anhydrase was [tris(2-benzimidazolylmethyl)amineZn(OH)₂], [tris(2-benzimidazolyl)amineZn(OH)₂](ClO₄)₂, and [tris(hydroxy-2-benzimidazolylmethyl)amineZn(OH)]ClO₄- 1.5H₂0 were also used to hydrate CO₂. (See Nakata et al., The Chemistry Letters, 1997, 991-992 and Echizen et al., Journal of Inorganic Biochemistry 2004, 98, 1347-1360).

For example, the carbonic anhydrase may be an alpha-carbonic anhydrase, a beta-carbonic anhydrase, a gamma-carbonic anhydrase, a delta-carbonic anhydrase, or an epsilon-carbonic anhydrase. Typically, the carbonic anhydrase is an alpha-carbonic anhydrase and further is a cytosolic carbonic anhydrase, a mitochondrial carbonic anhydrase, a secreted carbonic anhydrase, or a membrane-associated carbonic anhydrase.

More typically, the carbonic anhydrase is a mammalian carbonic anhydrase, a plant carbonic anhydrase, or a microbial carbonic anhydrase. Most typically, the carbonic anhydrase is a microbial carbonic anhydrase.

### Liquid Medium

The liquid medium comprises an aqueous mixture of one or more non- volatile CO₂ capture agents (i.e., proton accepting bases) that have a low heat of reaction for the overall CO₂ hydration reaction. The term "low heat of reaction", as used herein, refers to a molar heat of CO₂ absorption that is less than the molar heat of vaporization of water (for example, at the stripper liquid inlet temperature). A "non-volatile" capture agent, as used herein, refers to a proton accepting base formulation that has near zero vapor pressure in the absorber.

Preferably, the liquid medium comprises an organic or inorganic base. The base is a proton acceptor.

The base can be a metal hydroxide, a quaternary ammonium hydroxide, a metal carbonate, a quaternary ammonium carbonate, a quaternary ammonium alkoxide, a metal amide, a metal alkyl, a metal alkoxide, metal silanoate, an amine (primary, secondary, and tertiary), an amino acid, an alkanolamine, a conjugate base of a weak acid, or a combination thereof.

The metal hydroxide can include lithium hydroxide, sodium hydroxide, potassium hydroxide, rubidium hydroxide, cesium hydroxide, magnesium hydroxide, calcium hydroxide, strontium hydroxide, barium hydroxide, or a combination thereof. Also, ammonium hydroxide can be used in the aqueous liquid.

The metal carbonate can be lithium carbonate, sodium carbonate, potassium carbonate, rubidium carbonate, cesium carbonate, magnesium carbonate, calcium carbonate, strontium carbonate, barium carbonate, ammonium carbonate, a carbonate salt of an organic cation, or a combination thereof. For example, the carbonate salt of an organic cation can be a tetraalkyleammonium carbonate (e.g., tetramethylammonium carbonate, tetraethylammonium carbonate, tetrapropylammonium carbonate, tetrabutylammonium carbonate, tetrapentylammonium carbonate, or tetrahexylammonium carbonate) an alkyltrimethyl ammonium carbonate (e.g., ethyltrimethyl ammonium carbonate, propyltrimethyl ammonium carbonate, butyltrimethyl ammonium carbonate, pentyltrimethyl ammonium carbonate, hexyltrimethyl ammonium carbonate, hepyltrimethyl ammonium carbonate, octyltrimethyl ammonium carbonate, nonyltrimethyl ammonium carbonate, decyltrimethyl ammonium carbonate, dodecyltrimethyl ammonium carbonate, or undecyltrimethyl ammonium carbonate), an alkyltriethylammonium carbonate (e.g., methyltriethyl ammonium carbonate, propyltriethyl ammonium carbonate, butyltriethyl ammonium carbonate, pentyltriethyl ammonium carbonate, hexyltriethyl ammonium carbonate, hepyltriethyl ammonium carbonate, octyltriethyl ammonium carbonate, nonyltriethyl ammonium carbonate, decyltriethyl ammonium carbonate, dodecyltriethyl ammonium carbonate, or undecyltriethyl ammonium carbonate), an amino acid, or a combination thereof.

The quaternary ammonium hydroxide, quaternary ammonium carbonate, or quaternary ammonium alkoxide can be benzyltrimethylammonium hydroxide, choline hydroxide, diethyldimethylammonium hydroxide, dimethyldodecylethylammonium hydroxide, N,N,N,N',N',N'-hexabutylhexamethylenediammonium dihydroxide, hexadecyltrimethylammonium hydroxide, hexamethonium hydroxide, triethylmethylammonium hydroxide, tributylmethylammonium hydroxide, trihexyltetradecylammonium hydroxide, tetrapropylammonium hydroxide, tetrabutylammonium hydroxide, tetraoctadecylammonium hydroxide, methyltripropylammonium hydroxide, tetrabutylammonium ethoxide, tetraethylammonium hydroxide, tetrahexylammonium hydroxide, tetrakis(decyl)ammonium hydroxide, tetramethylammonium hydroxide, trimethylphenylammonium hydroxide, or a combination thereof.

The metal amide, metal alkoxide, or metal silanoate can be lithium tert-amoxide, lithium bis(trimethylsilyl)amide, lithium diethylamide, lithium dimethylamide, lithium diisopropylamide, sodium bis(trimethylsilyl)amide, potassium bis(trimethylsilyl)amide, lithium dicyclohexylamide, lithium trimethylsilanolate, sodium methoxide, potassium methoxide, lithium methoxide, sodium ethoxide, potassium ethoxide, lithium ethoxide, lithium isopropoxide, sodium tert-butoxide, potassium tert-butoxide, lithium tert-butoxide, sodium tert-pentoxide, potassium tert-pentoxide, magnesium ethoxide, magnesium di-tert-butoxide, sodium trimethylsilanolate, potassium trimethylsilanolate, or a combination thereof.

The amine can be a cyclic amine of 2-(2-chloro-6-fluorophenyl)ethylamine, 1,4-diazabicyclo[2.2.2]octane (DABCO^{®} 33-LV), 1,5-diazabicyclo[4.3.0]non-5-ene, 1,4-diazabicyclo[2.2.2]octane, 1,8-diazabicyclo[5.4.0]undec-7-ene, 4-(dimethylamino)pyridine, 2,6-lutidine, piperidine, 1,8-(dimethylamino)naphthalene, 2,2,6,6-tetramethylpiperidine, 2,8,9-triisobutyl-2,5,8,9-tetraaza- 1 - phosphabicyclo[3.3.3]undecane, tripelennamine, aniline, benzylamine, N-methyl aniline, imidazole, pyrrole, pyridine, morpholine, or a combination thereof.

The amine can be a primary amine, a secondary amine, a tertiary amine, or a combination thereof.

The primary amine can be methylamine, ethylamine, propylamine, isopropylamine, butylamine, iso-butylamine, sec-butylamine, tert-butylamine, pentylamine, iso-pentylamine, sec-pentylamine, tert-pentylamine, hexylamine, iso-hexylamine, sec-hexylamine, tert-hexylamine, ethylenediamine, (2-methylbutyl)amine, 2-aminopentane, 3-(tert-butoxy)propylamine, 2-amino-6-methylheptane, 1-ethylpropylamine, or a combination thereof.

Further, the secondary amine can be dimethylamine, diethylamine, dipropylamine, dibutylamine, dipentylamine, dihexylamine, methylethylamine, methylpropylamine, methylbutylamine, ethylpropylamine, ethylbutylamine, N-ethylmethylamine, N-isopropylmethylamine, N-butylmethylamine, N-ethylisopropylamine, N-tert-butylmethylamine, N-ethylbutylamine, 3 -isopropoxypropyl amine, chloro(diethylamino)dimethylsilane, 2,2'-(ethylenedioxy)bis(ethylamine), 1,3-bis(chloromethyl)-1,1,3,3-tetramethyldisilazane, N-tert-butylisopropylamine, N,N-diethyltrimethylsilylamine, di-sec-butylamine, or a combination thereof.

Additionally, the tertiary amine can be trimethylamine, triethylamine, tripropylamine, tributylamine, dimethylethylamine, dimethylpropylamine, dimethylbutylamine, diethylmethylamine, diethylpropylamine, diethylbutylamine, N,N-diisopropylmethylamine, N-ethyldiisopropylamine, N,N-dimethylethylamine, N,N-diethylbutylamine, 1,2-dimethylpropylamine, N,N-diethylmethylamine, N,N-dimethylisopropylamine, 1,3-dimethylbutylamine, 3,3-dimethylbutylamine, N,N-dimethylbutylamine, or a combination thereof.

The alkanolamine can be 2-amino-2-(hydroxymethyl)-1,3-propanediol (Trizma^{®} base), propanolamine, ethanolamine, diethanolamine, dimethylethanolamine, N-methylethanolamine, triethanolamine, or a combination thereof.

The conjugate base of a weak acid could be an acetate, a citrate, a succinate, an oxalate, a malate, a malonate, a phosphate, a phosphonate, a sulfate, a sulfamate, or a combination thereof wherein the counterion can be a positive ion such as an alkali metal, an alkaline earth metal, an ammonium cations, or a combination thereof.

The liquid medium can comprise an inorganic salt of carbonate (CO₃). For example, the liquid medium may comprise potassium carbonate (K₂CO₃).

The liquid medium can also comprise one or more amino acid salts, for example N-monosubstituted or N,N-disubstituted amino acid salts, which do not react significantly with unhydrated CO₂. The liquid medium may contain N-disubstituted (tertiary) aminoacid salts which have low heats of reaction, and/or it may contain sterically hindered N-monosubstituted amino acids salts with low heats of reaction.

Non-limiting examples of amino acid salts suitable for use with the processes described herein include salts of alanine, arginine, asparagine, asparagine, aspartic acid, cysteine, glutamic acid, glutamine, glycine, histidine, isoleucine, leucine, lysine, methionine, phenylalanine, proline, serine, threonine, tryptophan, tyrosine, valine, and mono- and di-substituted derivatives thereof.

The amino acid can be present in the form of an alkali metal salt thereof. For example, the amino acid may be activated with potassium hydroxide or other alkali hydroxide (e.g., lithium hydroxide or sodium hydroxide), which results in the amino acid salt.

Generally, the liquid medium can comprise a primary, secondary, or tertiary amino acid salt. Tertiary amino acid salts (i.e., di-substituted), in particular, exhibit favorable energetic properties with regard to both the absorption and desorption of CO₂.

For example, the liquid medium can comprise a sodium or a potassium salt of N,N-dimethylglycine.

The amino acid can be present as a salt where the cation will result in the greatest solubility of the amino acid salt and its CO₂ loaded product. For example, the amino acid may be present as the sodium or potassium salt thereof (e.g., the potassium salt of N,N-dimethyl glycine) .

The liquid medium can contain additives to enhance the life-time of the biocatalyst. The liquid medium may also contain additives to reduce the solubility of inhibitory heavy metal compounds.

Having described the invention in detail, it will be apparent that modifications and variations are possible without departing from the scope of the invention defined in the appended claims.

### EXAMPLES

The following non-limiting examples are provided to further illustrate the present invention.

### Example 1: Incorporation of Hydrophobic Additives in the Phosphate Buffer Diluted Synthesis and Entrapment of Carbonic Anhydrase in the Presence of 18-crown-6 in Polysilicate-Polysiloxane Particles

In a typical procedure, a 250 mL beaker was charged with 1.8 mL of a 150 mg/mL stock solution of carbonic anhydrase (carbonic anhydrase, NS81239, supplied by Novozymes A/S, Denmark).

Next a solution containing tetramethyl orthosilicate (3.3 mL, 21.7 mmol), an alkyl-trimethoxysilane (4.3 mmol) such as isobutyl (0.82 mL) or nonafluorohexyl (1.2 mL) trimethoxysilane, silanol-terminated poly(dimethyl siloxane) (average M_{w}= 550; 3.3 mL) and 18-crown-6 ether (0.9 g, 3.3 mmol) was added under vigorous stirring (or vortexing). Upon effective dispersion and subsequent emulsion formation, 45 mL of 50 mM potassium phosphate buffer (pH -7.2) containing 150 NH₄F catalyst was added to the stirring mixture.

The mixture turned opaque within 15-25 seconds and began to thicken after 45-60 seconds. With longer stirring, the mixture began to thin out enough to be able to transfer it.

It was transferred to a closed container to age for 22 hours at room temperature on a shaker plate.

After aging, the gel product was spray dried.

The spray dried powder was collected from the sample container and cured in a 95°C oven for 48 hours.

The powder was hydrated in 4 wt.% carbonate (pH -10) under stirring/agitation at 45°C until no dry powder was detected on top of the solution. To facilitate this initial hydration/washing process, acetone or ethanol (5-25 vol%) can be added. The particles were then collected via filtration and dried at 55°C overnight, and an aliquot of the hydration solution was collected to determine enzyme retention in the particles.

Alternatively, before drying, the particles were placed in an acetone/water mixture at room temperature under stirring/agitation for 24-48 hours and then collected via filtration.

The particles were the placed in pure acetone at room temperature under stirring/agitation for 24-48 hours. They were then collected via filtration and dried at 55°C overnight to remove any residual organic solvent.

These dried, washed particles were then rehydrated in the solvent of choice (potassium dimethyl glycine (KDMG) or carbonate) at 45°C in shaker/incubator for 2 days before testing for activity.

To characterize the buoyancy of the particles, a method was developed to monitor the rate separation of the particles from the solution (by floatation) using optical transmission. The method includes placing a well-mixed aliquot of the material, suspended in KDMG, into a 3 mL cuvette. The cuvette containing the sample is incubated in a temperature-controlled sample cell until it reaches the desired temperature. The sample is briefly removed, inverted several times to ensure adequate mixing, and immediately placed into a UV/Vis spectrometer. The spectrometer is setup to monitor light absorption/transmission at 450 nm over time. At the beginning of the run the biocatalyst materials obscure the light from reaching the detector. As the particles float to the top of the cuvette the solution clarifies allowing more light to pass through. Slowly separating particles yield flat profiles while quickly separating particles yield dynamic curves.

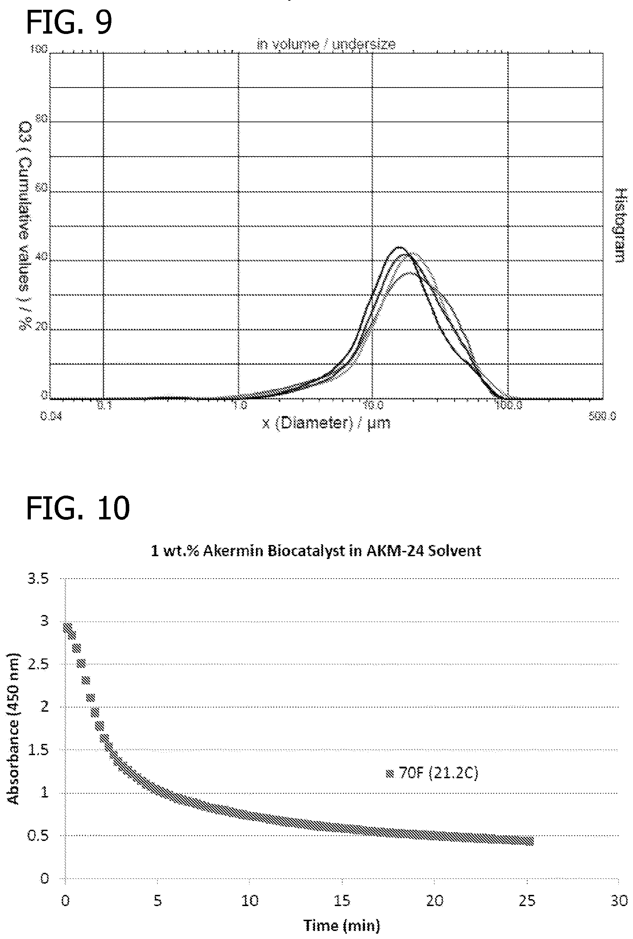

Figure 10 depicts the separation of the biocatalyst material over 30 minutes at 70°F. Figure 8 shows the effect of temperature on biocatalyst buoyancy. There is a direct correlation between temperature and the rate of particle separation in KDMG. The biocatalyst at 120°F and 140°F reach the same end point as the 70°F material in about one-third the time. It is believed that increased rate of separation is due to a reduction in the solution viscosity as the viscosity of KDMG at RT (20°C) is 4.0 cP, but drops to 1.6 cP at 60°C.

Another buoancy experiment used light absorption to track the clarification process. The same sample presented in Figure 8 demonstrated that a progressive clarification (decreased absorbance with time) was achieved within a few minutes, and this clarification process was enhanced with elevated solution temperatures.

### Example 2: Separation Using BRS-B System

A BRS system similar to the BRS-B system shown in Figure 3 was used to separate immobilized carbonic anhydrase particles from an aqueous solution. The immobilized carbonic anhydrase particles were prepared according to the procedure in Example 1 wherein 0.9 mL of carbonic anhydrase stock solution was used and 3.8 mL (26.0 mmol) of tetramethyl orthosilicate was used with no hydrophobic monomer. The feed stream for the separation contained 30.5 L KDMG and 0.71 wt.% immobilized carbonic anhydrase particles. The feed stream 1 was pumped through a cross-flow filter membrane unit 310 and the permeate was pumped out through stream 7 to result in approximately 29 L solution having no immobilized carbonic anhydrase particles detected. The concentrate was pumped through the reclaimed biocatalyst stream 5 and had -1.7 L solution and -14 wt.% immobilized carbonic anhydrase particles. This was a concentration of about 20 times as compared to the concentration of particles in the feed stream.

Further the particle size and distribution of the particle sizes of the immobilized carbonic anhydrase particles was measured in the feed stream and in the reclaimed biocatalyst stream 5 after separation. The results of those measurements are in Table 2.

**Table 2.**

| | Diameter at 10% (µm) | Diameter at 50% (µm) | Diameter at 90% (µm) |
|---|---|---|---|
| Feed Stream | 8 | 28 | 61 |
| Reclaimed Biocatalyst | 7 | 26 | 58 |

### Example 3: Separation Using a Dissolved Air Flotation (DAF) Technique

A process for CO₂ capture with Biocatalyst Recovery System was tested in an integrated system consisting of a Closed Loop Reactor and biocatalyst separator consisting of a combination of a Dissolved Air Floatation (DAF) unit and a Media Filter.

The BRS system enabled continuous biocatalyst management, including purge and make-up. A two-stage process was needed to achieve high recovery efficiency. The main separation step was based on dissolved air floatation (DAF) (passive separation), that took advantage of the low density biocatalyst particles and up to 99% removal of particles was achieved in this stage. A back-flushable media filter was also included to improve the overall efficiency to over 99.9%. The biocatalyst that was recovered from the media filter was back-flushed with rich solution- the use of rich solution for back-flush was enabled by the two-stage separation process- and then recycled to the front end of the DAF. The DAF technique was particularly advantageous because of its low capital cost and low energy requirements.

Moreover, it produced a biocatalyst concentrate thereby minimizing rich liquid carry-over and energy penalty. The biocatalyst concentrate was blended with lean solution and then fed to the top of the absorber column, completing the biocatalyst cycle.

Biocatalyst and white-water entered the trapezoidal chamber of the DAF. The biocatalyst concentrated in the upper portion of the tank while the liquid became progressively more clarified as it moved through the tank and was discharged through the valve on the lower section of the tank. The biocatalyst drained from the free surface into a triangular chamber before being discharged into a drain tube. Some surface vibration was necessary to move the biocatalyst from the main chamber. A mechanical wiper could have been used to harvest biocatalyst sludge from top of the DAF on a periodic basis.

The efficiency of the DAF was measured by withdrawing liquid samples from its feed and product liquid streams and analyzed for its biocatalyst content (each sample was quantitatively filtered, dried and the weight determined). With DAF feed mass flow of about 2 kg/minute and the biocatalyst concentration: about 0.5% up to 99% median efficiency of biocatalyst separation was confirmed. Due to the high separation efficiency in the DAF unit, the multi-media filters were back-flushed fewer that twice per day. Considering 95% efficiency in removing particles in the range of 5 to 10 microns in diameter and 99% efficiency for particles larger than 20 microns reported by the manufacture of the filter media (Nextsand^{®}) the combination efficiency of the DAF and the media filter is estimated to be more than 99.9%.

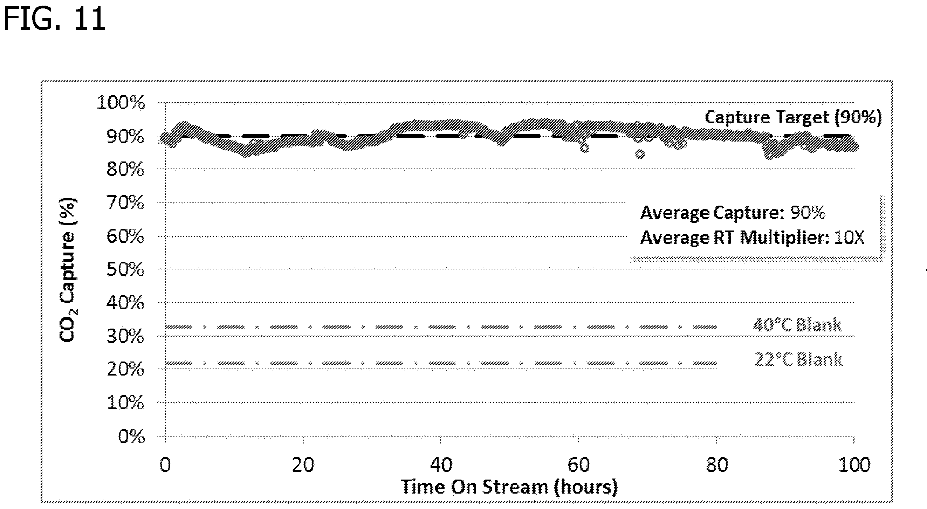

Testing of the performance of the integrated Closed Loop Reactor and the Biocatalyst Recovery System (DAF and Media filters) was conducted using 54 mm ID column absorber column with M500X packing at 15 SLPM of gas flow (13% CO₂) and 2 LPM liquid flow (30% AKM24). The results of the 100 hour test are presented in Fig. 11.

The results presented in Fig. 11 confirm a stable performance in the fully integrated system for 100 hours with the average CO₂ capture of 90%>. It must be emphasized that the performance of an equivalent system without the Biocatalyst Recovery System declines almost immediately due to inactivation of the biocatalyst upon exposure to the stripper temperature of about 105°C. Within the first 60 minutes the performance becomes virtually indistinguishable from the blank due to a complete enzyme inactivation.

It is clear that the Biocatalyst Recovery System described above provides a practical solution to the enzyme-catalyzed CO₂ capture that otherwise would not be of any use due to a rapid inactivation of the enzyme at temperatures greater than 100°C (reboiler and stripper temperatures).

When introducing elements of the present invention or the preferred embodiments(s) thereof, the articles "a", "an", "the" and "said" are intended to mean that there are one or more of the elements. The terms "comprising", "including" and "having" are intended to be inclusive and mean that there can be additional elements other than the listed elements.

In view of the above, it will be seen that the several objects of the invention are achieved and other advantageous results attained.

As various changes could be made in the above products and methods without departing from the scope of the invention, it is intended that all matter contained in the above description and shown in the accompanying drawings shall be interpreted as illustrative and not in a limiting sense.

## Claims

1. A process for removing CO₂ from a CO₂-containing gas, the process comprising: contacting a lean liquid medium with a CO₂-containing gas in an absorption zone in the presence of a biocatalyst that promotes the absorption of CO₂ from the gas phase into the liquid medium, thereby producing a rich liquid medium, wherein the biocatalyst comprises a carbonic anhydrase;
continuously separating the biocatalyst from the rich liquid medium to produce a separated biocatalyst and a separated rich liquid medium;
continuously introducing the separated rich liquid medium to a stripper to form a separated lean liquid medium and CO₂;
continuously introducing the separated biocatalyst to the separated lean liquid medium to form a lean liquid medium comprising the biocatalyst, and
reintroducing the lean liquid medium comprising the biocatalyst into the absorption zone and contacting it with the CO₂-containing gas;
**characterized in that** the separated biocatalyst has less than 7 gram rich liquid medium per gram biocatalyst based on a dry, salt free biocatalyst.

2. The process of claim 1, wherein the step of continuously separating the biocatalyst from the rich liquid medium comprises applying a flotation separation method, and optionally wherein the process comprises separating the biocatalyst from the rich liquid medium by removing floating particles from the surface of the rich liquid medium, using a floatation tank.

3. The process of claim 2, wherein the flotation separation method comprises introduction of gas microbubbles into the rich liquid medium.

4. The process of any one of claims 1 to 3, further comprising separating the biocatalyst from the rich liquid medium by filtering the biocatalyst.

5. The process of claim 4, wherein filtering of the biocatalyst further comprises a back flush of the filter with the lean liquid medium to produce the separated biocatalyst.

6. The process of claim 4 or 5, wherein the filtering of the biocatalyst is performed using a back-pulse or back-flush filter array, a cross flow membrane, a flow-through membrane, a sintered metal filter, a single media filter, a multimedia filter, or a combination thereof.

7. The process of claim 6, wherein the filtering of the biocatalyst is performed using a single media filter or a multimedia filter.

8. The process of any one of claims 2 or 3, wherein the biocatalyst has a density less than the rich liquid medium density.

9. The process of any one of claims 1 to 8, wherein the biocatalyst floats in the rich liquid medium.

10. The process of any one of claims 1 to 9, wherein the biocatalyst separation is performed with 90%, 91%, 92%, 93%, 94%, 95%, 96%, 97%, 98%, or 99% by weight separation efficiency.

11. The process of any one of claims 1 to 10, wherein the separated biocatalyst has less than 6 gram rich liquid medium per gram biocatalyst based on a dry, salt free biocatalyst.

12. The process of any one of claims 1 to 11, wherein the lean liquid comprises 0.1 to 3 wt.% biocatalyst.

13. The process of any one of claims 1 to 12, wherein the liquid medium comprises potassium carbonate.

14. The process of any one of claims 1 to 13, wherein at least a portion of the biocatalyst is immobilized by entrapment within a solid particulate material, and wherein the solid particulate material comprises a microparticle.

15. The process of claim 14, wherein the microparticle has an overall pore volume of at least 3 µL/g to 500 µL/g, wherein the microparticle has a surface area of from 1 m²/g to 400 m²/g, from 5 m²/g to 300 m²/g, from 10 to 150 m²/g, or from 15 to 100 m²/g, and wherein the microparticle has an average pore size of from 2 nm to 80 nm.

## Patentansprüche

1. Verfahren zum Entfernen von CO₂ aus einem CO₂-enthaltenden Gas, wobei das Verfahren umfasst: Kontaktieren eines mageren flüssigen Mediums mit einem CO₂-enthaltenden Gas in einer Absorptionszone in der Anwesenheit eines Biokatalysators, welcher die Absorption von CO₂ aus der Gasphase in das flüssige Medium unterstützt, wodurch ein angereichertes flüssiges Medium erzeugt wird, wobei der Biokatalysator eine Carboanhydrase umfasst;
kontinuierliches Trennen des Biokatalysators von dem angereicherten flüssigen Medium, um einen getrennten Biokatalysator und ein getrenntes angereichertes flüssiges Medium zu erzeugen;
kontinuierliches Einleiten des getrennten angereicherten flüssigen Mediums in einen Stripper, um ein getrenntes mageres flüssiges Medium und CO₂ zu erzeugen;
kontinuierliches Einleiten des getrennten Biokatalysators in das getrennte magere flüssige Medium, um ein mageres flüssiges Medium zu bilden, welches den Biokatalysator umfasst, und
Wiedereinleiten des mageren flüssigen Mediums, welches den Biokatalysator umfasst, in die Absorptionszone und Kontaktieren desselben mit dem CO₂-enthaltenden Gas;
**dadurch gekennzeichnet, dass** der getrennte Biokatalysator weniger als 7 Gramm angereichertes flüssiges Medium pro Gramm Biokatalysator, basierend auf einem trockenen, salzfreien Biokatalysator, aufweist.

2. Verfahren nach Anspruch 1, wobei der Schritt des kontinuierlichen Trennens des Biokatalysators von dem angereicherten flüssigen Medium ein Anwenden eines Flotation-Trenn-Verfahrens umfasst, und wobei das Verfahren optional ein Trennen des Biokatalysators von dem angereicherten flüssigen Medium durch Entfernen von schwimmenden Teilchen von der Oberfläche des angereicherten flüssigen Mediums unter Verwendung eines Flotationsbehälters umfasst.

3. Verfahren nach Anspruch 2, wobei das Flotation-Trenn-Verfahren eine Einleitung von Gas-Mikroblasen in das angereicherte flüssige Medium umfasst.

4. Verfahren nach einem der Ansprüche 1 bis 3, ferner umfassend Trennen des Biokatalysators von dem angereicherten flüssigen Medium durch Filtern des Biokatalysators.

5. Verfahren nach Anspruch 4, wobei das Filtern des Biokatalysators ferner eine Rückspülung des Filters mit dem mageren flüssigen Medium umfasst, um den getrennten Biokatalysator zu erzeugen.

6. Verfahren nach Anspruch 4 oder 5, wobei das Filtern des Biokatalysators unter Verwendung einer Rückimpuls- oder Rückspülung-Filteranordnung, einer Querstrom-Membran, einer Durchström-Membran, eines Filters aus gesintertem Metall, eines Einzel-Medienfilters, eines Multi-Medienfilters oder einer Kombination davon durchgeführt wird.

7. Verfahren nach Anspruch 6, wobei das Filtern des Biokatalysators unter Verwendung eines Einzel-Medienfilters oder eines Multi-Medienfilters durchgeführt wird.

8. Verfahren nach einem der Ansprüche 2 oder 3, wobei der Biokatalysator eine Dichte aufweist, welche geringer als eine Dichte des angereicherten flüssigen Mediums ist.

9. Verfahren nach einem der Ansprüche 1 bis 8, wobei der Biokatalysator in dem angereicherten flüssigen Medium schwimmt.

10. Verfahren nach einem der Ansprüche 1 bis 9, wobei die Biokatalysatortrennung mit 90, 91, 92, 93, 94, 95, 96, 97, 98 oder 99 Gewichts-% Trenneffizienz durchgeführt wird.

11. Verfahren nach einem der Ansprüche 1 bis 10, wobei der getrennte Biokatalysator weniger als 6 Gramm angereichertes flüssiges Medium pro Gramm Biokatalysator, basierend auf einem trockenen, salzfreien Biokatalysator, aufweist.

12. Verfahren nach einem der Ansprüche 1 bis 11, wobei das magere flüssige Medium 0,1 bis 3 Gew.-% Biokatalysator umfasst.

13. Verfahren nach einem der Ansprüche 1 bis 12, wobei das flüssige Medium Kaliumcarbonat umfasst.

14. Verfahren nach einem der Ansprüche 1 bis 13, wobei wenigstens ein Teil des Biokatalysators durch Einschluss innerhalb eines festen teilchenförmigen Materials immobilisiert wird, und wobei das feste teilchenförmige Material ein Mikroteilchen umfasst.

15. Verfahren nach Anspruch 14, wobei das Mikroteilchen ein Gesamtporenvolumen von wenigstens 3 µL/g bis 500 µL/g aufweist, wobei das Mikroteilchen eine Oberfläche von 1 m²/g bis 400 m²/g, von 5 m²/g bis 300 m²/g, von 10 bis 150 m²/g oder von 15 bis 100 m²/g aufweist, und wobei das Mikroteilchen eine durchschnittliche Porengröße von 2 nm bis 80 nm aufweist.

## Revendications

1. Procédé pour retirer du CO₂ d'un gaz contenant du CO₂, le procédé comprenant : la mise en contact d'un milieu liquide pauvre avec un gaz contenant du CO₂ dans une zone d'absorption en présence d'un biocatalyseur qui favorise l'absorption de CO₂ à partir de la phase gazeuse dans le milieu liquide, produisant ainsi un milieu liquide riche, dans lequel le biocatalyseur comprend une anhydrase carbonique ;
la séparation de manière continue du biocatalyseur du milieu liquide riche pour produire un biocatalyseur séparé et un milieu liquide riche séparé ;
l'introduction de manière continue du milieu liquide riche séparé dans un extracteur pour former un milieu liquide pauvre séparé et du CO₂ ;
l'introduction de manière continue du biocatalyseur séparé dans le milieu liquide pauvre séparé pour former un milieu liquide pauvre comprenant le biocatalyseur, et
la réintroduction du milieu liquide pauvre comprenant le biocatalyseur dans la zone d'absorption et sa mise en contact avec le gaz contenant du CO₂ ;
**caractérisé en ce que** le biocatalyseur séparé a moins de 7 grammes de milieu liquide riche par gramme de biocatalyseur sur la base d'un biocatalyseur sec, sans sel.

2. Procédé selon la revendication 1, dans lequel l'étape de séparation de manière continue du biocatalyseur du milieu liquide riche comprend l'application d'un procédé de séparation par flottation, et optionnellement dans lequel le procédé comprend la séparation du biocatalyseur du milieu liquide riche en retirant des particules flottantes de la surface du milieu liquide riche, en utilisant une cuve de flottation.

3. Procédé selon la revendication 2, dans lequel le procédé de séparation par flottation comprend l'introduction de microbulles gazeuses dans le milieu liquide riche.

4. Procédé selon l'une quelconque des revendications 1 à 3, comprenant en outre la séparation du biocatalyseur du milieu liquide riche en filtrant le biocatalyseur.

5. Procédé selon la revendication 4, dans lequel le filtrage du biocatalyseur comprend en outre un rinçage du filtre avec le milieu liquide pauvre pour produire le biocatalyseur séparé.

6. Procédé selon la revendication 4 ou 5, dans lequel le filtrage du biocatalyseur est réalisé en utilisant un réseau de filtres à pulsation inversée ou à rinçage, une membrane à flux croisé, une membrane à flux traversant, un filtre en métal fritté, un filtre à un seul milieu, un filtre à plusieurs milieux ou une combinaison de ceux-ci.

7. Procédé selon la revendication 6, dans lequel le filtrage du biocatalyseur est réalisé en utilisant un filtre à un seul milieu ou un filtre à plusieurs milieux.

8. Procédé selon l'une quelconque des revendications 2 ou 3, dans lequel le biocatalyseur a une densité inférieure à la densité de milieu liquide riche.

9. Procédé selon l'une quelconque des revendications 1 à 8, dans lequel le biocatalyseur flotte dans le milieu liquide riche.

10. Procédé selon l'une quelconque des revendications 1 à 9, dans lequel la séparation de biocatalyseur est réalisée avec une efficacité de séparation de 90 %, 91 %, 92 %, 93 %, 94 %, 95 %, 96 %, 97 %, 98 % ou 99 % en poids.

11. Procédé selon l'une quelconque des revendications 1 à 10, dans lequel le biocatalyseur séparé a moins de 6 grammes de milieu liquide riche par gramme de biocatalyseur sur la base d'un biocatalyseur sec, sans sel.

12. Procédé selon l'une quelconque des revendications 1 à 11, dans lequel le liquide pauvre comprend 0,1 à 3 % en poids de biocatalyseur.

13. Procédé selon l'une quelconque des revendications 1 à 12, dans lequel le milieu liquide comprend du carbonate de potassium.

14. Procédé selon l'une quelconque des revendications 1 à 13, dans lequel au moins une partie du biocatalyseur est immobilisée par un piégeage dans un matériau particulaire solide, et dans lequel le matériau particulaire solide comprend une microparticule.

15. Procédé selon la revendication 14, dans lequel la microparticule a un volume poreux global d'au moins 3 µl/g à 500 µl/g, dans lequel la microparticule a une superficie de 1 m²/g à 400 m²/g, de 5 m²/g à 300 m²/g, de 10 à 150 m²/g ou de 15 à 100 m²/g, et dans lequel la microparticule a une taille de pores moyenne de 2 nm à 80 nm.
